# EUROPÄISCHE PATENTSCHRIFT

(11) **EP 4 251 596 B1**
(45) Veröffentlichungstag und Bekanntmachung des Hinweises auf die Patenterteilung: **31.07.2024**
(21) Anmeldenummer: 22702218.3
(22) Anmeldetag: 21.01.2022
(51) Int. Cl.: C07C 7/08, C07C 11/08, C07C 2/08, C07C 11/02

(54) **VERFAHREN ZUR ABTRENNUNG VON BUTENEN AUS C4-KOHLENWASSERSTOFFSTRÖMEN MIT ANGESCHLOSSENER OLIGOMERISIERUNG**
METHOD FOR THE SEPARATION OF BUTENES FROM C4 HYDROCARBON FLOWS WITH CONNECTED OLIGOMERIZATION
PROCÉDÉ DE SÉPARATION DES BUTÈNES DES FLUX D'HYDROCARBURE C4 COMPRENANT L'OLIGOMÉRISATION

(30) Priorität: 27.01.2021 EP 21153663
(43) Veröffentlichungstag der Anmeldung: 04.10.2023
(73) Patentinhaber: Evonik Oxeno GmbH & Co. KG, 45772 Marl (DE)
(72) Erfinder: LUTZE, Philip, 46535 Dinslaken (DE); PEITZ, Stephan, 45739 Oer-Erkenschwick (DE); RIX, Armin Matthias, 45770 Marl (DE); SIX, Tanita Valèrie, 44309 Dortmund (DE); SCHRÖDER, Moritz, 48153 Münster (DE); PAUL, Niklas, 45770 Marl (DE)
(74) Vertreter: Evonik Patent Association
(86) Internationale Anmeldenummer: PCT/EP2022/051378
(87) Internationale Veröffentlichungsnummer: WO 2022/161871

(56) Entgegenhaltungen:
- US-A1- 2006 264 686
- US-A1- 2014 124 358

## Beschreibung

Die vorliegende Erfindung betrifft ein Verfahren zur Abtrennung von Butenen aus C4-Kohlenwasserstoffströmen, die neben den Butenen auch Butane enthält, durch Extraktivdestillation mit einem geeigneten Lösemittel und eine anschließende adiabate Oligomerisierung. Das erfindungsgemäße Verfahren zeichnet sich durch eine Wärmeintegration aus, mit der die Wärme des Lösemittels zur Erwärmung und/oder zumindest teilweiser Verdampfung verschiedener Ströme genutzt wird.

Die Trennung von Butan-Buten-Gemischen mittels Extraktivdestillation ist an sich bekannt. Dabei wird ein aprotisches Lösemittel (z. B. N-Methyl-2-pyrrolidon (NMP) oder Acetonitril (ACN)) eingesetzt, um die relative Flüchtigkeit der Alkane gegenüber den Alkenen zu erhöhen. In einer Extraktivdestillationskolonne, dem Absorber, werden bevorzugt die Butene im Lösemittel gelöst, die Butane werden als Kopfprodukt abgetrennt. Das beladene Lösemittel wird anschließend in einer Stripkolonne, dem Desorber, bei erhöhter Temperatur und/oder reduziertem Druck von den Butenen befreit, die als Kopfprodukt in angereicherter Form gewonnen werden. Das von Butenen befreite Lösemittel wird dann zur Extraktivdestillation zurückgefahren.

Aufgrund des hohen Lösemittel-zu-Feed-Verhältnisses kommt der Wärmeintegration eine hohe Bedeutung für die Wirtschaftlichkeit des Verfahrens zu. Im Sumpf des Desorbers fällt heißes Lösemittel an, dessen Energieinhalt auf verschiedene Weise genutzt werden kann. In der US 2014/0124358 A1 wird ein Verfahren zur selektiven Extraktion von Olefinen vorgestellt, welches die Aufgabe der Wärmeintegration lösen soll. Darin wird vorgeschlagen, den Energiegehalt des heißen Lösemittel zur Erwärmung eines Seitenstroms aus dem Desorber, zur Erwärmung des Sumpfprodukts des Absorbers, das zum Desorber geleitet wird, zur Erwärmung von einem oder mehreren Seitenströmen des Absorbers, und zur Vorwärmung des Feedstroms zu nutzen.

Weiterhin werden im Stand der Technik oft nur Verfahren zur Trennung von Butan-Buten-Gemischen offenbart, die eine möglichst hohe Ausbeute bei der Trennung zum Ziel haben. Es kann jedoch gewünscht sein, dass je nach dem Einbau einer solchen Trennung in einem chemischen Verbund noch nachgeschaltete Prozesse mit butenhaltigen C4-Strömen versorgt werden, beispielsweise eine adiabate Oligomerisierung.

Die im Stand der Technik vorgeschlagene Lösung kann die Aufgabe einer möglichst vollständigen Rückgewinnung der im System vorhandenen Energieströme jedoch entweder nicht ganz oder nur durch relativ komplexe Aufbauten erfüllen. Eine weitere Aufgabe war die Bereitstellung eines butanhaltigen Produktstroms, der noch eine signifikante Menge an Butenen enthält und in einem nachgeschalteten Prozessen, beispielsweise einer Oligomerisierung, eingesetzt werden kann.

Die Aufgabe der vorliegenden Erfindung bestand deshalb darin ein Verfahren bereitzustellen, bei dem eine verbesserte, bestenfalls maximale Energierückgewinnung erzielt wird und das anlagentechnisch weniger aufwändig ist.

Diese Aufgabe kann durch die in Anspruch 1 vorgeschlagene Ausführung des Verfahrens gelöst werden. Bevorzugte Ausführungsformen sind in den Unteransprüchen angegeben. Das erfindungsgemäße Verfahren ist ein Verfahren zur Abtrennung von Butenen aus einem C4-Kohlenwasserstoffstrom, der zumindest Butene und Butane enthält, durch Extraktivdestillation mit einem Lösemittel, wobei das Verfahren die folgenden Schritte umfasst:
a. Zumindest teilweises Verdampfen des flüssigen C4-Kohlenwasserstoffstroms in einem Feedverdampfer, Zuführen des gasförmigen C4-Kohlenwasserstoffstroms und Zuführen des flüssigen Lösemittels, vorzugsweise NMP, oberhalb des C4-Kohlenwasserstoffstroms zu einem Absorber, in dem der C4-Kohlenwasserstoffstrom und das Lösemittel miteinander in Kontakt gebracht werden, wodurch überwiegend Butene aus dem C4-Kohlenwasserstoffstrom in das Lösemittel übergehen, wobei das so beladene Lösemittel, vorzugsweise NMP, in einem Flüssigkeitssammler des Absorbers gesammelt und durch einen Absorberverdampfer gefahren und dann unterhalb des Flüssigkeitssammlers in den Sumpf des Absorbers geleitet wird, wodurch überwiegend Butane aus dem beladenen Lösemittel, vorzugsweise NMP, ausgegast werden, wobei das beladene Lösemittel, vorzugsweise NMP, anschließend als Sumpfstrom zu einem Desorber geführt wird und wobei am Kopf des Absorbers ein im Vergleich zum eingesetzten C4-Kohlenwasserstoffstrom an Butanen angereicherter Strom anfällt, der noch mindestens 5 Gew.-% an Butenen, vorzugsweise mindestens 10 Gew.-% an Butenen, besonders bevorzugt mindestens 15 Gew.-% an Butenen, enthält;
b. Zuführen des beladenen Lösemittels, vorzugsweise NMP, zu dem Desorber, in dem im Vergleich mit dem Absorber eine erhöhte Temperatur und bevorzugt ein geringerer Druck vorliegt und in dem die Butene vom Lösemittel, vorzugsweise NMP, getrennt werden, wodurch am Kopf des Desorbers ein an Butenen angereicherter Strom und im Sumpf des Desorbers das zumindest teilweise von Butenen befreite Lösemittel, vorzugsweise NMP, anfällt, wobei das zumindest teilweise von Butenen befreite Lösemittel, vorzugsweise NMP, in einem Flüssigkeitssammler des Desorbers gesammelt und durch einen Desorberverdampfer gefahren und dann unterhalb des Flüssigkeitssammlers in den Sumpf des Desorbers geleitet wird, wodurch noch im Lösemittel vorhandene Butene ausgegast werden, und wobei das Lösemittel, vorzugsweise NMP, anschließend als Sumpfstrom zum Absorber zurückgeführt wird;

wobei die Wärme des als Sumpfstrom des Desorbers abgenommen Lösemittels, vorzugsweise des NMPs, zur Wärmeintegration verwendet wird, in dem die Wärme des Lösemittels, vorzugsweise des NMPs, in jeweils mindestens einem Wärmetauscher für die Vorwärmung des zum Desorber geführten beladenen Lösemittels, vorzugsweise NMP, zur Verdampfung im Absorberverdampfer und zur Verdampfung des flüssigen C4-Kohlenwasserstoffstroms eingesetzt wird und wobei
der an Butanen angereicherte Strom zu einer adiabaten Oligomerisierung geführt wird, um die im Strom vorhandenen Butene zu oligomerisieren.

Ein Vorteil des vorliegenden Verfahrens ist der relativ einfache Aufbau der Wärmeintegration, die dennoch eine effiziente Energierückgewinnung ermöglicht. Ein weiterer Vorteil ist, dass die Trennung im Absorber nicht so scharf gefahren wird und deshalb noch signifikante Mengen an Butenen im butanhaltigen Produktstrom vorhanden sind. Dadurch kann eine angeschlossene adiabate Oligomerisierung betrieben werden, wodurch werthaltige C8-Olefine produziert werden können.

Durch die Wärmeintegration wird dem Lösemittel Wärme entzogen. Der Grund dafür ist nicht nur, dass damit andere Ströme oder Kolonnen geheizt werden sollen, sondern in erster Linie die Abkühlung des Lösemittels für die Absorption. Die Absorption der Butene (hier: Schritt a) findet meistens bei einer geringeren Temperatur statt als die Desorption (hier: Schritt b). Wird dem Lösemittel bei der Wärmeintegration ausreichend Wärme entzogen, weist also eine geeignete Temperatur auf, kann das Lösemittel direkt in den Absorber gefahren werden. Es ist jedoch auch denkbar, dass das Lösemittel trotz der vorliegenden Wärmeintegration noch nicht die richtige Temperatur aufweist. In so einem Fall kann das Lösemittel nach der Wärmeintegration und vor dem Eintritt in den Absorber durch einen Restkühler geleitet werden, um auf eine geeignete Temperatur gekühlt zu werden.

Wärme ist eine Prozessgröße. Die zu- oder abgeführte Wärme entspricht der Änderung der Inneren Energie abzüglich der verrichteten Arbeit. Bei den in dieser Erfindung verwendeten Begriffe Wärme, Wärmetransport und Wärmeintegration wird diese Definition stets zu Grunde gelegt.

Das vorliegende Verfahren betrifft die Abtrennung von Butenen aus butenhaltigen C4-Kohlenwasserstoffströmen. Diese Ströme enthalten üblicherweise neben den Butenen auch Alkane (n-Butan, Isobutan). Der Begriff Butane wird im Rahmen der vorliegenden Erfindung dabei so verstanden, dass er - sofern nichts anderes bezeichnet ist - sowohl n-Butan als auch Isobutan meint. Im erfindungsgemäßen Verfahren können daher alle C4-Kohlenwasserstoffströme eingesetzt werden, die zumindest Butene und Butane enthalten, sofern die Mengen, in denen die Butene und/oder Butane vorhanden sind, eine wirtschaftliche Durchführung des Verfahrens zulassen. In einer bevorzugten Ausführungsform der vorliegenden Erfindung besteht der eingesetzte C4-Kohlenwasserstoffstrom im Wesentlichen, d.h. zu mehr als 98 Gew.-%, vorzugsweise zu mehr als 99 Gew.-% aus Butanen und Butenen. Die entsprechenden Ströme können auch Verunreinigungen oder andere Kohlenwasserstoffe, wie 1,3-Butadien oder C5-Kohlenwasserstoffe, in geringen Mengen enthalten.

In dem erfindungsgemäßen Extraktionsverfahren wird ein flüssiges Lösemittel eingesetzt, in dem sich vorrangig die Butene des eingesetzten, gasförmigen C4-Kohlenwasserstoffstroms lösen. Geeignete Lösemittel sind aprotische Lösemittel, beispielsweise N-Methyl-2-pyrrolidon (NMP). Das erfindungsgemäße Verfahren wird vorzugsweise mit NMP als Lösemittel durchgeführt. In einer weiterhin bevorzugten Ausführungsform der vorliegenden Erfindung enthält das Lösemittel Wasser, insbesondere im Bereich von 1 bis 10 Gew.-%, vorzugsweise von 4 bis 9 Gew.-%, jeweils bezogen auf die Gesamtmenge an Lösemittel.

Als Absorber können insbesondere Füllkörperkolonnen dienen, die mindestens zwei Füllkörperbetten aufweisen. Solche Kolonnen sind dem Fachmann grundsätzlich bekannt. Oberhalb des ersten Füllkörperbettes befindet sich vorzugsweise eine Rückwaschzone mit mehreren theoretischen Böden, um das in die Gasphase mitgerissene Lösemittel zurückzuhalten. Oberhalb der Rückwaschzone ist der Kopf des Absorbers, wo ein im Vergleich zum eingesetzten C4-Kohlenwasserstoffstrom an Butanen angereicherter Strom anfällt. Unter dem letzten Füllkörperbett würde der erfindungsgemäße Flüssigkeitssammler angeordnet sein, worunter sich der Sumpf des Absorbers befindet. Der exakte Aufbau des Absorbers hängt von verschiedenen Parametern ab und ist in gewisser Hinsicht variabel.

Das flüssige Lösemittel wird räumlich betrachtet oberhalb des Einlasses für den C4-Kohlenwasserstoffstroms zu dem Absorber gegeben. In einer bevorzugten Ausführungsform wird das Lösemittel oberhalb des ersten Füllkörperbettes und der C4-Kohlenwasserstoffstrom in ein oder mehrere Füllkörperbetten unterhalb des ersten Füllkörperbettes zum Absorber gegeben. Das flüssige Lösemittel wird im Absorber nach unten rieseln und mit dem dampfförmigen C4-Kohlenwasserstoffstrom in Kontakt gebracht, wodurch ein Teil des C4-Kohlenwasserstoffstroms, der überwiegend Butene enthält, in das Lösemittel übergeht. Vorliegend wird die Abtrennung erfindungsgemäß nicht so scharf gefahren. Das bedeutet, dass es das Ziel ist, dass noch mindestens 5 Gew.-% Butene, vorzugsweise mindestens 10 Gew.-% Butene, besonders bevorzugt mindestens 15 Gew.-% Butene im butanhaltigen Strom am Kopf des Absorbers vorhanden sind. Eine Möglichkeit, um das zu erreichen, ist, dass im Vergleich zu einer Trennung, bei der die Butene fast vollständig in das Lösemittel übergehen, die Menge an Lösemittel reduziert wird, die auf den Absorber geschickt wird. Dadurch können weniger Butene in das Lösemittel übergehen.

Das beladene Lösemittel läuft im Absorber nach unten und wird in einem geeigneten Flüssigkeitssammler, insbesondere einem Kaminboden, gesammelt. Die Temperatur des im Flüssigkeitssammler anfallenden beladenen Lösemittels beträgt vorzugsweise zwischen 40 und 90°C, besonders bevorzugt zwischen 45 und 65 °C. Vom Flüssigkeitssammler wird das beladene Lösemittel abgenommen, durch einen Absorberverdampfer gefahren und dann unterhalb des Flüssigkeitssammlers in den Sumpf des Absorbers geleitet, wodurch überwiegend Butane aus dem beladenen Lösemittel ausgegast werden. Der Absorberverdampfer ist vorzugsweise ein Once-Through-Verdampfer, bei dem das beladene Lösemittel nur einmal durch den Verdampfer geleitet wird. Dadurch lassen sich möglichst niedrige Temperaturen realisieren, wodurch Fouling verhindert werden kann. Zudem wird die treibende Temperaturdifferenz vergrößert, was eine noch effizientere Energieausnutzung des NMP-Stroms ermöglicht. Der Absorberverdampfer kann auch mehrstufig ausgestaltet sein, d. h. es können mehrere Wärmetauscher bzw. mehrere Verdampfer vorhanden sein, die zum Absorberverdampfer gehören.

Im Sumpf verbleibt dann das vorwiegend mit Butenen beladene Lösemittel, welches von dort abgenommen und als Sumpfstrom zum Desorber geführt wird. Die Temperatur im Sumpfstrom des Absorbers, der zum Desorber geführt wird, beträgt dabei vorzugsweise zwischen 70 und 130 °C, besonders bevorzugt zwischen 85 und 120 °C.

Am Kopf des Absorbers fällt dann insbesondere ein im Vergleich zum eingesetzten C4-Kohlenwasserstoffstrom an Butanen angereicherter Strom an der noch mindestens 5 Gew.-% an Butenen, vorzugsweise mindestens 10 Gew.-% an Butenen, besonders bevorzugt mindestens 15 Gew.-% an Butenen, enthält. Der Kopfdruck im Absorber kann dabei zwischen 3 und 7 bar absolut, vorzugsweise zwischen 4 und 6,5 bar absolut betragen. Der an Butanen angereicherte Strom kann zusätzlich Wasser enthalten, welches aus dem Lösemittel stammt. Dieses Wasser kann in einem nachfolgenden Schritt abgetrennt werden. Dabei wird der an Butanen angereicherte Strom am Kopf des Absorbers entnommen und einer ein- oder mehrstufigen Kondensation unterworfen, wobei ein wasserhaltiger Strom und ein butanhaltiger Produktstrom auskondensiert werden. Diese beiden Ströme können in einer geeigneten Vorrichtung, beispielsweise einem Euter, voneinander getrennt werden. Der vom butanhaltigen Produktstrom abgetrennte wasserhaltige Strom kann je nach seiner Zusammensetzung zum Absorber oder zum Desorber geführt und/oder teilweise aus dem Verfahren ausgeschleust werden.

Der so aus der Kondensation erhaltene butanhaltige Produktstrom kann noch geringe Mengen an Wasser enthalten, insbesondere in einer Menge von bis zu 1500 Gew.-ppm, bezogen auf die Gesamtzusammensetzung des butanhaltigen Produktstroms.

Je nach den Anforderungen an den erhaltenen butanhaltigen Produktstrom kann es erforderlich sein, dass der butanhaltige Produktstrom nach der Kondensation einer Trocknung, vorzugsweise in einer Trocknungskolonne, unterworfen wird, um das noch enthaltene Wasser abzutrennen. Vorzugsweise weist der butanhaltige Produktstrom nach der Trocknung eine maximale Menge an Wasser von 50 Gew.-ppm, vorzugsweise von 25 Gew.-ppm auf. Das bei der Trocknung anfallende Wasser kann zur Kondensation am Absorber zurückgeführt werden.

Der an Butanen angereichter Strom oder sofern eine Kondensation vorliegt der butanhaltige Produktstrom werden anschließend zu einer adiabaten Oligomerisierung geführt, um die im Strom vorhandenen Butene zu oligomerisieren. Die adiabate Oligomerisierung wird in einem oder mehreren parallelen oder in Reihe geschalteten Reaktoren durchgeführt. Der Begriff adiabat ist hierbei so zu verstehen, dass der oder die Reaktoren nicht aktiv gekühlt werden. Nach der Reaktion in dem einen oder in den mehreren Reaktoren kann der erhaltenen Produktstrom aufgearbeitet werden, um die erhaltenen Oligomere von nicht umgesetzten Edukten abzutrennen. Eine solche Trennung erfolgt vorzugsweise mittels Destillation. Die nicht umgesetzten Edukte können zu dem oder den Reaktoren zurückgeführt werden.

Die Bedingungen einer adiabaten Oligomerisierung sind dem Fachmann grundsätzlich geläufig. Die adiabate Oligomerisierung wird vorzugsweise bei einer Temperatur in einem Bereich von 80 bis 110 °C, besonders bevorzugt 90 bis 100 °C durchgeführt. Der Druck beträgt mindestens 20 bar absolut, vorzugsweise mindestens 25 bar absolut, besonders bevorzugt mindestens 30 bar absolut. Als Katalysatoren sind grundsätzlich alle geeigneten Katalysatoren eingesetzt werden. Bevorzugt sind hier Nickel enthaltende Katalysatoren, die Nickel und ein Trägermaterial umfassen. Als Reaktoren sind Festbettreaktoren geeignet.

Das im Sumpf des Absorbers abgenommene und vorwiegend mit Butenen beladenen Lösemittel wird dem Desorber zugeführt. Das beladene Lösemittel kann dazu beispielsweise mittels einer Pumpe zum Desorber geführt werden. Im Sumpf des Desorbers liegt im Vergleich zum Sumpf des Absorbers eine erhöhte Temperatur und bevorzugt ein geringerer Druck vor. Die Temperatur im Sumpf des Desorbers beträgt vorzugsweise zwischen 120 und 200 °C, weiterhin bevorzugt zwischen 130 und 195 °C. Der Kopfdruck im Desorber kann zwischen 1 und 6 bar absolut, vorzugsweise zwischen 2 und 5 bar absolut betragen. Durch die gegenüber dem Absorber erhöhte Temperatur und den vorzugsweise geringeren Druck werden die Butene und optional noch enthaltenen Butane zumindest teilweise aus dem Lösemittel entfernt. In einer bevorzugten Ausführungsform enthält das zumindest teilweise von Butenen befreite Lösemittel bis zu 5000 Gew.-ppm an Butenen, besonders bevorzugt 100 bis 900 Gew.-ppm an Butenen. Das zumindest teilweise von Butenen befreite Lösemittel läuft im Desorber nach unten und wird in einem Flüssigkeitssammler des Desorbers gesammelt. Von dort wird das zumindest teilweise von Butenen befreite Lösemittel durch einen Desorberverdampfer gefahren und dann unterhalb des Flüssigkeitssammlers, insbesondere einem Kaminboden, in den Sumpf des Desorbers geleitet, wodurch noch im Lösemittel vorhandene Butene ausgegast werden. Der Desorberverdampfer ist vorzugsweise ein Once-Through-Verdampfer, bei dem das zumindest teilweise von Butenen befreite Lösemittel nur einmal durch den Verdampfer geleitet wird. Dadurch lassen sich möglichst niedrige Temperaturen realisieren, wodurch Fouling verhindert werden kann. Der Desorberverdampfer kann auch mehrstufig ausgestaltet sein, d. h. es können mehrere Wärmetauscher vorhanden sein, die zum Desorberverdampfer gehören. Im Sumpf verbleibt dann das von Butenen befreite Lösemittel, welches von dort abgenommen, als Sumpfstrom zum Absorber geführt und dort wieder als Lösemittel für die Absorption von Butenen eingesetzt wird.

Das von Butenen befreite Lösemittel kann bevor es zum Absorber geführt wird teilweise oder vollständig einer Regeneration unterworfen werden, wodurch Verunreinigungen, beispielsweise die vorgenannt im eingesetzten C4-Kohlenwasserstoffstrom vorhandenen und/oder bei den Temperaturen im Desorber aus den Butenen gebildeten Nebenprodukte wie oligomere oder polymere Verbindungen, aus dem Lösemittel, vorzugsweise dem NMP, entfernt werden. Die Regeneration wird vorzugsweise so durchgeführt, dass das von Butenen befreite Lösemittel in einen Behälter gefahren und bei einem Druck von weniger als 500 mbar absolut, weiterhin bevorzugt von weniger als 200 mbar absolut und einer Temperatur zwischen 100 und 150 °C verdampft wird. An den Behälter kann eine Kolonne angeschlossen sein. Durch die Regeneration werden insbesondere Schwersieder abgetrennt. Wird nur ein Teil des von Butenen befreiten Lösemittels einer Regeneration unterworfen, wird der regenerierte Teil des Lösemittels anschließend mit dem nicht regenerierten Lösemittel vereint und zum Absorber zurückgeführt.

Am Kopf des Desorbers fällt dann insbesondere ein im Vergleich zu dem eingesetzten C4-Kohlenwasserstoffstrom an Butenen angereicherte Strom an. Dieser an Butenen angereicherte Strom kann zusätzlich Wasser enthalten, welches aus dem Lösemittel stammt. Dieses Wasser kann in einem nachfolgenden Schritt abgetrennt werden. Dabei wird der an Butenen angereichte Strom am Kopf des Desorbers entnommen und einer ein- oder mehrstufigen Kondensation unterworfen, wobei ein wasserhaltiger Strom, der neben Wasser auch noch Reste an Organik enthalten kann, und ein butenhaltiger Produktstrom auskondensiert werden. Diese beiden Ströme können in einer geeigneten Vorrichtung, beispielsweise einem Euter, voneinander getrennt werden. Das vom butenhaltigen Produktstrom abgetrennte wasserhaltige Strom kann anschließend zurück zum Desorber geführt werden. Möglich ist auch eine Ausschleusung des ganzen oder von Teilen des wasserhaltigen Stroms, um die Organik zu entfernen.

In einer bevorzugten Ausführungsform der vorliegenden Erfindung ist die Kondensation des am Kopf des Desorbers abgenommenen an Butenen angereicherten Stroms zweistufig ausgestaltet, wobei in einer ersten Stufe ein wasserhaltiger Strom auskondensiert wird, das dann zum Desorber zurückgeführt wird, und in der zweiten Stufe der butenhaltige Produktstrom auskondensiert wird. Es kann jedoch auch sein, dass in der zweiten Stufe auch noch vorhandenes Wasser auskondensiert. Dieses restliche Wasser kann über eine geeignete Vorrichtung, beispielsweise einen Euter, vom butenhaltigen Produktstrom abgetrennt werden.

Der aus der Kondensation erhaltene butenhaltige Produktstrom enthält vorzugsweise weniger als 20 Gew.-%, weiterhin bevorzugt weniger als 16 Gew.-% an Butanen bezogen auf die Gesamtzusammensetzung des butenhaltigen Produktstroms. Der aus der Kondensation erhaltene butenhaltige Produktstrom weist stattdessen vorzugsweise einen Butengehalt von mindestens 70 Gew.-%, weiterhin bevorzugt von mindestens 75 Gew.-%, besonders bevorzugt von mindestens 86 Gew.-% bezogen auf die Gesamtzusammensetzung des butenhaltigen Produktstroms auf.

Ein kennzeichnendes Merkmal der vorliegenden Erfindung ist die Wärmeintegration unter Verwendung der Wärme des Lösemittels auf dem Weg vom Desorber zum Absorber und des Heißkondensats, welches im Desorberverdampfer anfällt. Die Wärme des als Sumpfstrom des Desorbers abgenommen Lösemittels, vorzugsweise des NMPs, wird erfindungsgemäß zur Wärmeintegration verwendet, in dem die Wärme des Lösemittels in jeweils mindestens einem Wärmetauscher für die Vorwärmung des zum Desorber geführten beladenen Lösemittels zur Verdampfung im Absorberverdampfer und zur Verdampfung des flüssigen C4-Kohlenwasserstoffstroms eingesetzt wird.

In einer bevorzugten Ausgestaltung der vorliegenden Erfindung wird die Vorwärmung des zum Desorber geführten beladenen Lösemittels zweistufig durchgeführt, wobei eine erste eine Wärmeübertragung auf das zum Desorber geführten beladenen Lösemittel in einem Wärmetauscher, beispielsweise einen Rohrbündelwärmetauscher, und eine zweite Wärmeübertragung auf das zum Desorber geführten beladenen Lösemittel in einem Kettleverdampfer erfolgt. Eine solche Ausgestaltung hat den Vorteil, dass bei der vorgenannten bevorzugten Ausgestaltung die Wärme, die in beiden Stufen, d. h. im Wärmetauscher und im Kettleverdampfer auf das beladene Lösemittel übertragen wird, aus dem als Sumpfstrom des Desorbers abgenommen Lösemittel als Wärmeträgermedium stammt. Die Verwendung eines Kettleverdampfers hätte auch den Vorteil, dass in der Leitung zum Desorber ein geringerer Vordruck herrschen könnte. Normalerweise ist ein hoher Vordruck notwendig, um das Verdampfen in der Rohrleitung zu verhindern, was zu Problemen bis zum Bersten der Rohrleitung führen könnte. Ein weiterer Vorteil ist, dass die Temperaturbelastung begrenzt und dadurch sichergestellt wird, dass die Temperaturdifferenz zur Wärmeübertragung ausreichend groß ist bzw. bleibt.

Erfindungsgemäß wird das zumindest teilweise von Butenen befreite Lösemittel, vorzugsweise NMP, in einem Flüssigkeitssammler des Desorbers gesammelt und durch einen Desorberverdampfer gefahren, wodurch noch im Lösemittel vorhandene Butene ausgegast werden können. Die Wärme zur Verdampfung im Desorberverdampfer kann dabei in einem Wärmetauscher durch Wärmeübertragung von einem geeigneten Wärmeträgermedium eingetragen werden. Das Wärmeträgermedium kann insbesondere Heizdampf sein, der als Mittel- oder Hochdruckdampf eingesetzt wird. Als Heizdampf ist ein Mitteldruckdampf bevorzugt, welcher eine Temperatur von 150 bis 270 °C, vorzugsweise von 160 bis 250 °C aufweist. Der Mitteldruckdampf weist vorzugsweise einen Druck von 15 bis 30 bar absolut, besonders bevorzugt von 17 bis 25 bar absolut auf. Als Heizdampf kann auch ein Dampf mit einem Druck von > 30 bar absolut eingesetzt werden. Ein solcher Heizdampf kann auch als Hochdruckdampf bezeichnet werden.

Der zur Verdampfung eingesetzte Heizdampf kann im Wärmetauscher zumindest teilweise kondensieren, wodurch ein Heißkondensat bei einem Druck von 10 bis 20 bar absolut, vorzugsweise 12 bis 17 bar absolut und einer Temperatur von 150 bis 210 °C, vorzugsweise 160 bis 200 °C anfällt. Nach dem Wärmetauscher ist vorzugsweise ein Kondensatbehälter angeordnet, in dem das Heißkondensat vom Dampf getrennt werden kann. In dem Kondensatbehälter liegt vorzugsweise ein geringerer Druck vor als im Wärmetauscher auf der Heizdampfseite. Aufgrund des geringeren Drucks kann ein Teil des Heißkondensats verdampfen, wodurch der gesamte Dampf, d. h. der nicht kondensierte Anteil des Heizdampfes und das im Kondensatbehälter durch Druckentspannung verdampfte Heißkondensat, im Kondensatbehälter als Niederdruckdampf anfällt. Niederdruckdampf weist vorliegend vorzugsweise einen Druck von mehr als 0 bar und weniger als 10 bar absolut auf. Die Temperatur des Niederdruckdampfs beträgt vorzugsweise 100 bis 180 °C.

Der dort anfallende Niederdruckdampf enthält noch Energie, die in keinem bekannten Verfahren ausgenutzt wird. Aus energetischer und wirtschaftlicher Sicht ist das jedoch nicht sinnvoll. Diese Energie kann jedoch in einer bevorzugten Ausgestaltung der vorliegenden Erfindung genutzt werden. Dazu kann der zur Verdampfung im Desorberverdampfer eingesetzte Heizdampf mittels eines, vorzugsweise regelbaren Dampfstrahlers (Thermokompressors) zur Verfügung gestellt werden. Der Thermokompressor wird dann sowohl mit dem eingesetzten Heizdampf, der beispielsweise aus einem entsprechenden Dampfnetz stammt, hier insbesondere der bevorzugt eingesetzte Mitteldruckdampf, als auch mit dem Niederdruckdampf aus dem Kondensatbehälter gespeist, wodurch ein Mischdampf entsteht, der dementsprechend das Wärmeträgermedium für den Desorberverdampfer ist. Der Mischdampf ist in dieser Ausgestaltung demnach der Heizdampf. Ein solcher Dampfstrahler ist so ausgestaltet, dass er mit einem Treibdampf betrieben wird und durch einen Unterdruck (Staudruck im Dampfstrahler) Saugdampf aus einem Behälter ansaugen kann, wodurch dann der Mischdampf entsteht, der als Wärmeträgermedium eingesetzt wird. Der Treibdampf ist im vorliegenden Fall der Heizdampf bzw. der Mitteldruckdampf, mit dem der Niederdruckdampf als Saugdampf aus dem Kondensatbehälter angesaugt und mit dem Treibdampf vermischt wird.

Der Vorteil einer solchen Ausgestaltung ist offensichtlich. Die Energie des im Kondensatbehälter anfallenden Niederdruckdampfes kann genutzt und damit Energie und Kosten gespart werden. Eine solche Verfahrensweise kann auch aus einem anderen Grund vorteilhaft sein. Der eingesetzte Dampfstrahler kann dahingehend regelbar sein, dass die Mengen von Mitteldruck- und Niederdruckdampf, beispielsweise in Abhängigkeit von bestimmten Prozessparametern, eingestellt werden können. Die Saugdampfmenge stellt sich dabei über die Treibdampfmenge ein. Die Mengen von Mitteldruck- und Niederdruckdampf können beispielsweise in Abhängigkeit von der Temperatur im Desorber eingestellt werden.

Eine weiterhin bevorzugte Ausgestaltung kann auch dann vorliegen, wenn der Desorber einen Seitenverdampfer aufweist. In einem solchen Fall kann das Wärmeträgermedium, welches für den Seitenverdampfer eingesetzt wird, der Mischdampf aus dem Dampfstrahler sein, während im Desorberverdampfer Mitteldruckdampf als Heizdampf eingesetzt wird. Das Heißkondensat aus dem Desorberverdampfer und dem Seitenverdampfer werden dann entsprechend den obigen Ausführungen zu einem Kondensatbehälter geleitet. Der dort anfallende Niederdruckdampf wird dann im Dampfstrahler verwendet, dessen Mischdampf im Seitenverdampfer eingesetzt wird. Der Vorteil dieser Variante ist, dass das anfallende Heißkondensat weiter entspannt werden könnte, um eine größere Niederdruckdampfmenge bereitstellen zu können.

Das vorliegend beschriebene Verfahren kann in chemischen Verbünden, die insbesondere eine Oligomerisierung und optional eine Hydroformylierung umfassen, eingesetzt werden. Dabei ist es möglich, dass die Abtrennung von Butenen nach dem erfindungsgemäßen Verfahren an verschiedenen Stellen im Verbund eingesetzt wird. Es ist auch möglich, wenn die erfindungsgemäße Abtrennung von Butenen an mehreren Stellen innerhalb eines chemischen Verbundes vorhanden ist. Möglich ist beispielsweise, dass das hier beschriebene Verfahren zu Beginn eines solchen Verbunds eingesetzt wird. Der eingesetzte C4-Kohlenwasserstoffstrom kann dann insbesondere ein Crack-C4, ein Raffinat 1, ein Raffinat 2 oder eine Mischung daraus sein. Sofern Crack-C4 und/oder das Raffinat 2 eingesetzt werden, kann vor dem erfindungsgemäßen Abtrennverfahren eine Crack-C4-Hydrierung, in der Butadien selektiv hydriert wird, oder eine Butadienabtrennung, bei der Butadien extraktiv mit einem Lösemittel wie NMP oder Nitrilen entfernt wird, erfolgen, um den Gehalt an Butadien zu reduzieren. Nach einer extraktiven Butadienabtrennung und vor der erfindungsgemäßen Abtrennung kann eine Hydroisomerisierung angeordnet sein, um die Trennaufgabe beim erfindungsgemäßen Verfahren zu erleichtern, da 1-Buten in 2-Buten umgewandelt wird, was vom Lösemittel in der Regel besser aufgenommen wird.

Wird das Abtrennverfahren zu Beginn des Verbunds eingesetzt, kann der erhaltene Produktstrom einer MTBE-Synthese zugeführt werden und vorzugsweise anschließend sukzessive eine 1-Butenabtrennung, eine Oligomerisierung und eine oder mehrere Hydroformylierung(en) auf die aufgereinigten Oligomere erfolgen. Eine Hydroformylierung kann sowohl mit dem Produktstrom aus der Oligomerisierung erfolgen, wodurch zum Beispiel aus Di-n-butenen nach anschließender Hydrierung INA (Isononanol) oder aus Tributenen ITDA (Isotridecanal) hergestellt werden kann, als auch mit den nicht umgesetzten Butenen der Oligomerisierung, wodurch nach anschließender Aldolkondensation und nachfolgender Hydrierung 2-PH (2-Propylheptanol) hergestellt werden kann. Mit den nicht umgesetzten Butenen aus der Oligomerisierung könnte ggf. auch anstatt einer Hydroformylierung eine weitere Oligomerisierung betrieben werden. Die Bedingungen der einzelnen Verfahrensschritte sind dem Fachmann geläufig. Die einzelnen Verfahrensschritte können weitere Schritte wie beispielsweise die Abtrennung der Produkte oder die Aufarbeitung der resultierenden Ströme enthalten, sind hier jedoch nicht explizit genannt. Das erfindungsgemäße Abtrennverfahren kann aber auch an jeder anderen Stelle eines solchen Verbunds eingefügt werden.

In einer Ausführungsform der vorliegenden Erfindung wird der beim erfindungsgemäßen Abtrennverfahren eingesetzte C4-Kohlenwasserstoffstrom einer MTBE-Synthese nach der Abtrennung von MTBE entnommen und der butenhaltige Produktstrom anschließend einer 1-Butenabtrennung zugeführt, wonach sukzessive eine Oligomerisierung und eine oder mehrere Hydroformylierung(en) zur anschließenden Herstellung von 2-PH, ITDA und/oder INA erfolgen. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird der beim erfindungsgemäßen Abtrennverfahren eingesetzte C4-Kohlenwasserstoffstrom einer 1-Butenabtrennung entnommen und der butenhaltige Produktstrom anschließend einer Oligomerisierung zugeführt, wonach eine oder mehrere Hydroformylierung(en) zur anschließenden Herstellung von 2-PH, ITDA und/oder INA erfolgen. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird der beim erfindungsgemäßen Abtrennverfahren eingesetzte C4-Kohlenwasserstoffstrom einer Oligomerisierung entnommen und der butenhaltige Produktstrom anschließend einer Hydroformylierung zur anschließenden Herstellung von 2-PH zugeführt. Die einzelnen Verfahrensschritte können weitere Schritte wie beispielsweise die Abtrennung der Produkte oder die Aufarbeitung der resultierenden Ströme enthalten, sind hier jedoch nicht explizit genannt.

In einer weiteren Ausführungsform der vorliegenden Erfindung wird das erfindungsgemäße Abtrennverfahren am Ende des Verbunds eingesetzt. In dem Fall wird der eingesetzte C4-Kohlenwasserstoffstrom einer der Hydroformylierung sich anschließenden 2-PH-Herstellung entnommen. Der aus dem erfindungsgemäßen Abtrennverfahren sodann erhaltene butenhaltige Produktstrom kann in diesem Fall zurückgeführt werden und an einer geeigneten Stelle im Verbund, beispielsweise zur 1-Butenabtrennung, zur Oligomerisierung oder einer oder mehrere Hydroformylierung(en) eingesetzt werden. Dadurch kann die Effizienz des gesamten Verbunds gesteigert werden, da selbst nach Durchlaufen des letzten Verfahrensschrittes im Verbund noch bis zu 20 Gew.-% Butene vorhanden sein können.

Der butanhaltige Produktstrom kann unabhängig von der Stelle im Verbund, in der das erfindungsgemäße Abtrennverfahren angeordnet ist, beispielsweise einer adiabaten Oligomerisierung, einer Hydrierung der noch vorhandenen Butene oder einer n/-iso-Splittung der Butane, bei der n-Butan und Isobutan voneinander getrennt werden, zugeführt werden. Die n/iso-Splittung kann auch nach einer adiabaten Oligomerisierung erfolgen. Möglich wäre auch eine Einbindung des butanhaltigen Produktstroms vor der Oligomerisierung in einem oben beschriebenen Verbund aus MTBE-Synthese, 1-Butenabtrennung, Oligomerisierung und einer Hydroformylierung.

In einer besonders bevorzugten Ausführungsform der vorliegenden Erfindung kann die zur n/iso-Splittung erforderliche Energie zumindest teilweise durch Wärmeintegration mit der ersten Stufe einer zweistufigen Kondensation am Kopf des Desorbers erfolgen. Das hat den Vorteil, dass die Energie, die in der Kondensation anfällt, genutzt wird und nicht wie im Stand der Technik einfach an die Umgebung abgegeben wird.

Die vorliegende Erfindung wird nachfolgend anhand von Abbildungen erläutert. Die Abbildungen dienen der Veranschaulichung, sind aber nicht einschränkend zu verstehen.

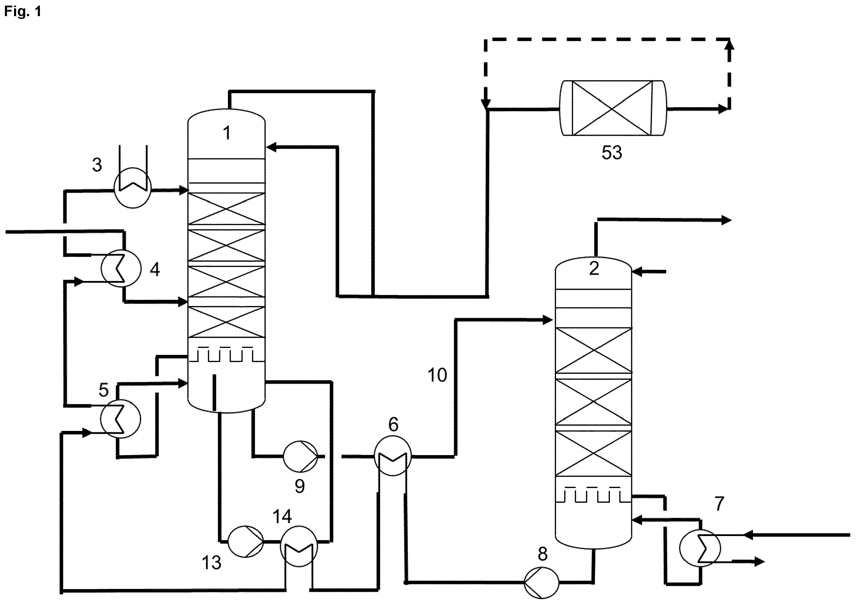

Fig. 1 zeigt die grundsätzliche Ausgestaltung der vorliegenden Erfindung. Der flüssige C4-Kohlenwasserstoffstrom wird über einen Wärmetauscher (4) verdampft und in den Absorber (1) geleitet. Das Lösemittel wird über einen Restkühler (3) - wenn notwendig - auf die gewünschte Temperatur gebracht und ebenfalls in den Absorber geleitet, wobei der Einlass räumlich gesehen oberhalb des Einlasses für den C4-Kohlenwasserstoffstrom ist, im vorliegenden Fall oberhalb des ersten Füllkörperbettes. Am Kopf des Absorbers (1) fällt der an Butanen angereicherte Strom an, der abgenommen und zu einer adiabaten Oligomerisierung (53) geleitet wird. Der aus der Oligomerisierung erhaltenen Strom kann teilweise rezykliert werden, was hier nur angedeutet ist. Im Sumpf des Absorbers (1) wird das beladene Lösemittel gesammelt, was in der Abbildung durch den Kaminboden angedeutet ist. Dort wird zumindest ein Teil des beladenen Lösemittels abgenommen und über einen Absorberverdampfer (5) zum Sumpf des Absorbers (1) geführt. Aus dem Sumpf des Absorbers (1) wird das beladene Lösemittel abgenommen und mittels einer Pumpe (9) über den Wärmetauscher (6) zur Vorwärmung des beladenen Lösemittels zum Desorber geleitet (2), wo die im Lösemittel vorhandenen Butene vom Lösemittel getrennt werden.

Am Kopf des Desorbers fällt der an Butenen angereicherte Strom an. Dieser Strom kann einer ein- oder mehrstufigen Kondensation unterworfen werden, was in der Abbildung nicht gezeigt ist. Lediglich ein möglicher Rückführstrom ist angedeutet. Im Sumpf des Desorbers (2) wird das zumindest teilweise von Butenen befreite Lösemittel gesammelt, was in der Abbildung durch den Kaminboden angedeutet ist. Dort wird zumindest ein Teil des beladenen Lösemittels abgenommen und über einen Desorberverdampfer (7) zum Sumpf des Desorbers geführt. Aus dem Sumpf des Desorbers (2) wird dann das von Butenen befreite Lösemittel abgenommen und mittels einer Pumpe (8) über den Wärmetauscher (6) zur Vorwärmung des beladenen Lösemittels, den Absorberverdampfer (5), den Wärmetauscher (4) zur Verdampfung des C4-Kohlenwasserstoffstroms und den Restkühler (3) zum Absorber zurückgeführt.

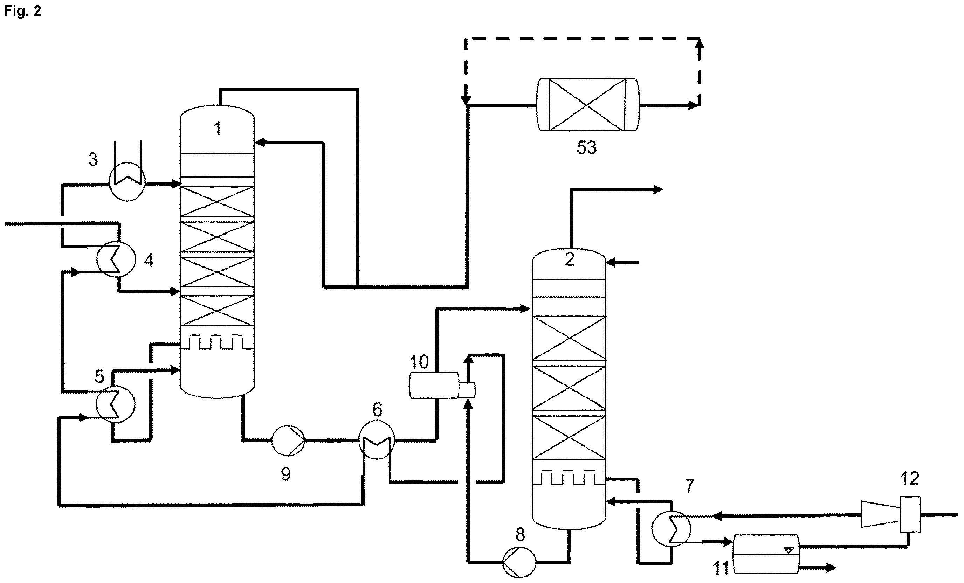

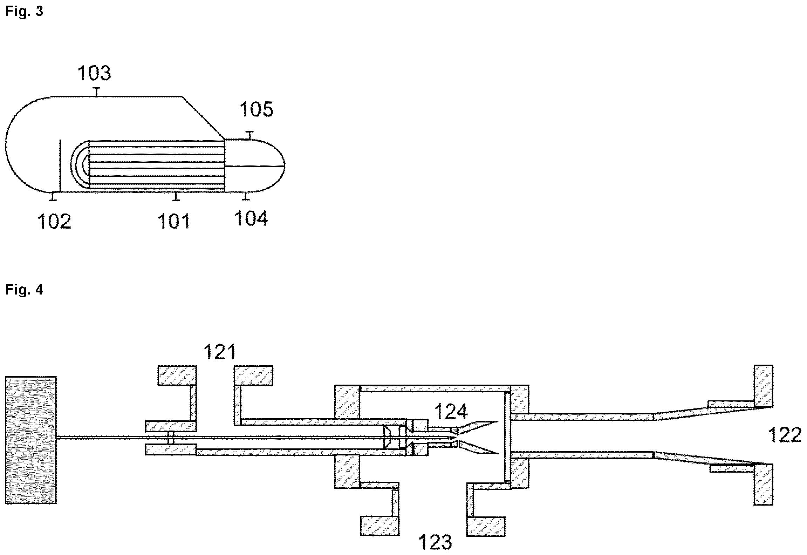

Fig. 2 zeigt eine bevorzugte Ausführungsform der vorliegenden Erfindung, bei der die Vorwärmung des zum Desorber (2) geführten beladenen Lösemittels zweistufig, d. h. mit einem zusätzlichen Kettleverdampfer (10) durchgeführt wird und bei der am Desorberverdampfer (7) ein Dampfstrahler (12) vorhanden ist. Dieser Dampfstrahler wird mit dem regulären Heizdampf, also beispielsweise dem Mitteldruckdampf aus dem Dampfnetz, und dem Niederdruckdampf, der im Kondensatbehälter (11) anfällt, gespeist, wodurch ein Mischdampf entsteht, der dann als Heizdampf zum Desorberverdampfer (7) verwendet wird. Die Funktionsweise eines Kettleverdampfers wird bei der Beschreibung von Fig. 3 und die Funktionsweise eines Dampfstrahlers wird bei der Beschreibung von Fig. 4 erläutert. Alles weitere entspricht den vorherigen Ausführungen zu Fig. 1.

Fig. 3 zeigt den schematischen Aufbau eines Kettleverdampfers (10). Über den Feedstutzen (101) auf der Mantelseite wird der flüssige Feed in den Verdampfer gefahren. Der flüssige Feed wird teilweise im Kettleverdampfer verdampft und über den Gasstutzen (103) auf der Mantelseite in den Desorber gefahren. Der Anteil des Feeds, der nicht verdampft wird, läuft über ein Wehr ab und über den Produktstutzen (102) auf der Mantelseite als flüssiges Produkt in den Desorber (2). Auf der Rohrseite wird das heiße Sumpfprodukt des Desorbers (2) als Heizmedium verwendet, das über den Eintrittsstutzen (104) durch die Rohre gefahren wird und am Austrittsstutzen (105) wieder austritt.

Fig. 4 zeigt den schematischen Aufbau eines Dampfstrahlers (12). Der Treibdampf (121) ist hier der Heizdampf, insbesondere der Mitteldruckdampf aus dem Dampfnetz. Der Saugdampf (123) ist der Niederdruckdampf aus dem Kondensatbehälter. Beide werden über die Regeleinheit (124) vermischt und über den Ausgang als Mischdampf (122) zum Desorberverdampfer (7) geführt. Über die Regeleinheit können die Mengen von Treibdampf und Saugdampf eingestellt werden, wodurch Druck und Temperatur des Mischdampfes und damit die mögliche Heizleistung beeinflusst werden können.

## Patentansprüche

1. Verfahren zur Abtrennung von Butenen aus einem C4-Kohlenwasserstoffstrom, der zumindest Butene und Butane enthält, durch Extraktivdestillation mit einem Lösemittel, wobei das Verfahren die folgenden Schritte umfasst:
a. Zumindest teilweises Verdampfen des flüssigen C4-Kohlenwasserstoffstroms in einem Feedverdampfer, Zuführen des gasförmigen C4-Kohlenwasserstoffstroms und Zuführen des flüssigen Lösemittels oberhalb des C4-Kohlenwasserstoffstroms zu einem Absorber, in dem der C4-Kohlenwasserstoffstrom und das Lösemittel miteinander in Kontakt gebracht werden, wodurch überwiegend Butene aus dem C4-Kohlenwasserstoffstrom in das Lösemittel übergehen, wobei das so beladene Lösemittel in einem Flüssigkeitssammler des Absorbers gesammelt und durch einen Absorberverdampfer gefahren und dann unterhalb des Flüssigkeitssammlers in den Sumpf des Absorbers geleitet wird, wodurch überwiegend Butane aus dem beladenen Lösemittel ausgegast werden, wobei das beladene Lösemittel anschließend als Sumpfstrom zu einem Desorber geführt wird und wobei am Kopf des Absorbers ein im Vergleich zum eingesetzten C4-Kohlenwasserstoffstrom an Butanen angereicherter Strom anfällt, der noch mindestens 5 Gew.-% an Butenen, vorzugsweise mindestens 10 Gew.-% an Butenen, besonders bevorzugt mindestens 15 Gew.-% an Butenen enthält;
b. Zuführen des beladenen Lösemittels zu dem Desorber, in dem im Vergleich mit dem Absorber eine erhöhte Temperatur und bevorzugt ein geringerer Druck vorliegt und in dem die Butene vom Lösemittel getrennt werden, wodurch am Kopf des Desorbers ein an Butenen angereicherter Strom und im Sumpf des Desorbers das zumindest teilweise von Butenen befreite Lösemittel anfällt, wobei das zumindest teilweise von Butenen befreite Lösemittel in einem Flüssigkeitssammler des Desorbers gesammelt und durch einen Desorberverdampfer gefahren und dann unterhalb des Flüssigkeitssammlers in den Sumpf des Desorbers geleitet wird, wodurch noch im Lösemittel vorhandene Butene ausgegast werden, und wobei das Lösemittel anschließend als Sumpfstrom zum Absorber zurückgeführt wird;
**dadurch gekennzeichnet, dass** die Wärme des als Sumpfstrom des Desorbers abgenommen Lösemittels zur Wärmeintegration verwendet wird, in dem die Wärme des Lösemittels in jeweils mindestens einem Wärmetauscher für die Vorwärmung des zum Desorber geführten beladenen Lösemittels, zur Verdampfung im Absorberverdampfer und zur Verdampfung des flüssigen C4-Kohlenwasserstoffstroms eingesetzt wird, und dass
der an Butanen angereicherte Strom zu einer adiabaten Oligomerisierung geführt wird, um die im Strom vorhandenen Butene zu oligomerisieren.

2. Verfahren nach Anspruch 1, wobei das eingesetzte Lösemittel NMP ist.

3. Verfahren nach Anspruch 1 oder 2, wobei das Lösemittel bzw. das NMP Wasser enthält und der Wassergehalt zwischen 1 und 10 Gew.-%, vorzugsweise zwischen 4 und 9 Gew.-% liegt.

4. Verfahren nach einem der vorhergehenden Ansprüche, wobei der an Butenen angereicherte Strom, der am Kopf des Desorbers anfällt, zusätzlich Wasser enthält, welches aus dem Lösemittel stammt.

5. Verfahren nach Anspruch 4, wobei der an Butenen angereichter Strom am Kopf des Desorbers entnommen und einer Kondensation unterworfen wird, wobei Wasser und butenhaltiger Produktstrom auskondensiert und voneinander getrennt werden und das Wasser zurück zum Absorber geführt wird.

6. Verfahren nach Anspruch 5, wobei der aus der Kondensation erhaltene butenhaltige Produktstrom einen Butengehalt von mindestens 70 Gew.-%, vorzugsweise mindestens 75 Gew.-%, besonders bevorzugt mindestens 86 Gew.-% bezogen auf die Gesamtzusammensetzung des butenhaltigen Produktstroms, aufweist.

7. Verfahren nach einem der vorhergehenden Ansprüche, wobei die Temperatur im Sumpfstrom des Absorbers, der zum Desorber geführt wird, 70 und 130 °C, vorzugsweise 85 bis 120 °C beträgt.

8. Verfahren nach einem der vorhergehenden Ansprüche, wobei die Temperatur im Sumpf des Desorbers zwischen 120 und 200 °C, vorzugsweise 130 und 195 °C beträgt.

9. Verfahren nach einem der vorhergehenden Ansprüche, wobei der Kopfdruck im Desorber zwischen 1 und 6 bar absolut, vorzugsweise 2 bis 5 bar absolut beträgt.

10. Verfahren nach einem der vorhergehenden Ansprüche, wobei die Vorwärmung des zum Desorber geführten beladenen Lösemittels zweistufig durchgeführt wird, wobei eine erste eine Wärmeübertragung auf das Lösemittel in einem Wärmetauscher und eine zweite Wärmeübertragung auf das Lösemittel in einem Kettleverdampfer erfolgt.

11. Verfahren nach einem der vorhergehenden Ansprüche, wobei die Wärme zur Verdampfung im Desorberverdampfer durch Wärmeaustausch in einem Wärmetauscher mit einem geeigneten Wärmeträgermedium, insbesondere Heizdampf, eingetragen wird.

12. Verfahren nach einem der vorhergehenden Ansprüche, wobei der eingesetzte Heizdampf im Wärmetauscher zumindest teilweise kondensiert und dadurch ein Heißkondensat bei einem Druck von 10 bis 20 bar absolut, vorzugsweise 12 bis 17 bar absolut und einer Temperatur von 150 bis 200 °C, vorzugsweise 160 bis 190 °C anfällt, das zu einem Kondensatbehälter geleitet wird.

13. Verfahren nach Anspruch 12, wobei im Kondensatbehälter ein geringerer Druck vorliegt als im Wärmetauscher und dadurch ein Teil des Heißkondensats wieder verdampft, wodurch der gesamte Dampf als Niederdruckdampf anfällt.

14. Verfahren nach einem der vorhergehenden Ansprüche, wobei der Heizdampf für den Desorberverdampfer mittels eines Dampfstrahlers, der mit Mitteldruckdampf und dem im Kondensatbehälter anfallenden Niederdruckdampf gespeist wird, zur Verfügung gestellt wird.

## Claims

1. Process for separating butenes from a C4-hydrocarbon stream which contains at least butenes and butanes by extractive distillation with a solvent, wherein the process comprises the steps of:
a. at least partially evaporating the liquid C4-hydrocarbon stream in a feed evaporator, supplying the gaseous C4-hydrocarbon stream and supplying the liquid solvent above the C4-hydrocarbon stream to an absorber in which the C4-hydrocarbon stream and the solvent are contacted with one another to transfer predominantly butenes from the C4-hydrocarbon stream to the solvent, wherein the thus-laden solvent is collected in a liquid collector of the absorber, passed through an absorber evaporator and then passed into the bottom of the absorber below the liquid collector to outgas predominantly butanes from the laden solvent, wherein the laden solvent is subsequently passed to a desorber as bottoms stream and wherein a stream enriched in butanes relative to the employed C4-hydrocarbon stream and still containing at least 5% by weight of butenes, preferably at least 10% by weight of butenes, particularly preferably at least 15% by weight of butenes, is obtained at the top of the absorber;
b. supplying the laden solvent to the desorber which is at an elevated temperature and preferably a lower pressure relative to the absorber and in which the butenes are separated from the solvent to obtain at the top of the desorber a stream enriched in butenes and in the bottom of the desorber the solvent at least partially freed of butenes, wherein the solvent, at least partially freed of butenes, is collected in a liquid collector of the desorber and passed through a desorber evaporator and then passed into the bottom of the desorber below the liquid collector to outgas any butenes remaining in the solvent and wherein the solvent is subsequently recycled to the absorber as bottoms stream;
**characterized in that** the heat of the solvent withdrawn as a bottoms stream of the desorber is used for heat integration by employing the heat of the solvent in at least one respective heat exchanger for preheating the laden solvent passed to the desorber, for evaporation in the absorber evaporator and for evaporation of the liquid C4-hydrocarbon stream and **in that**
the stream enriched in butanes is passed to an adiabatic oligomerization to oligomerize the butenes present in the stream.

2. Process according to Claim 1, wherein the employed solvent is NMP.

3. Process according to Claim 1 or 2, wherein the solvent/the NMP contains water and the water content is between 1% and 10% by weight, preferably between 4% and 9% by weight.

4. Process according to any of the preceding claims, wherein the stream enriched in butenes obtained at the top of the desorber additionally contains water originating from the solvent.

5. Process according to Claim 4, wherein the stream enriched in butenes is withdrawn at the top of the desorber and subjected to a condensation, wherein water and butene-containing product stream are condensed out and separated from one another and the water is returned to the absorber.

6. Process according to Claim 5, wherein the butene-containing product stream obtained from the condensation has a butene content of at least 70% by weight, preferably at least 75% by weight, particularly preferably at least 86% by weight, based on the total composition of the butene-containing product stream.

7. Process according to any of the preceding claims, wherein the temperature in the bottoms stream of the absorber, which is passed to the desorber, is 70°C and 130°C, preferably 85°C to 120°C.

8. Process according to any of the preceding claims, wherein the temperature in the bottom of the desorber is between 120°C and 200°C, preferably 130°C and 195°C.

9. Process according to any of the preceding claims, wherein the head pressure in the desorber is between 1 and 6 bar absolute, preferably 2 to 5 bar absolute.

10. Process according to any of the preceding claims, wherein the preheating of the laden solvent passed to the desorber is performed in two stages, wherein a first heat transfer to the solvent is effected in a heat exchanger and a second heat transfer to the solvent is effected in a kettle evaporator.

11. Process according to any of the preceding claims, wherein the heat for evaporation in the desorber evaporator is introduced in a heat exchanger by heat transfer with a suitable heat transfer medium, in particular heating steam.

12. Process according to any of the preceding claims, wherein the employed heating steam undergoes at least partial condensation in the heat exchanger, thus generating a hot condensate at a pressure of 10 to 20 bar absolute, preferably 12 to 17 bar absolute, and a temperature of 150°C to 200°C, preferably 160°C to 190°C, which is passed to a condensate container.

13. Process according to Claim 12, wherein the pressure in the condensate container is lower than in the heat exchanger, thus causing a portion of the hot condensate to be re-evaporated, as a result of which the combined steam is obtained as low pressure steam.

14. Process according to any of the preceding claims, wherein the heating steam for the desorber evaporator is provided using a steam ejector supplied with medium pressure steam and the low pressure steam obtained in the condensate container.

## Revendications

1. Procédé de séparation de butènes à partir d'un flux hydrocarboné en C4, qui contient au moins des butènes et des butanes, par distillation extractive à l'aide d'un solvant, le procédé comprenant les étapes suivantes :
a. évaporation au moins partielle du flux hydrocarboné liquide en C4 dans un évaporateur à alimentation, fourniture du flux hydrocarboné gazeux en C4 et fourniture du solvant liquide au-dessus du flux hydrocarboné en C4 à un absorbeur, dans lequel le flux hydrocarboné en C4 et le solvant sont amenés en contact l'un avec l'autre, suite à quoi les butènes passent principalement à partir du flux hydrocarboné en C4 dans le solvant, le solvant ainsi chargé étant collecté dans un collecteur de liquide de l'absorbeur et transporté à travers un évaporateur de l'absorbeur puis guidé sous le collecteur de liquide dans le fond de l'absorbeur, suite à quoi les butanes sont principalement dégazés à partir du solvant chargé, le solvant chargé étant ensuite guidé en tant que flux de fond vers un désorbeur et un flux enrichi en butanes par rapport au flux hydrocarboné en C4 utilisé étant obtenu en tête de l'absorbeur, lequel flux contenant encore au moins 5% en poids de butènes, de préférence au moins 10% en poids de butènes, de manière particulièrement préférée au moins 15% en poids de butènes ;
b. fourniture du solvant chargé à un désorbeur, dans lequel, par rapport à l'absorbeur, la température est plus élevée et de préférence la pression plus basse et dans lequel les butènes sont séparés du solvant, suite à quoi on obtient en tête du désorbeur un flux enrichi en butènes et on obtient, dans le fond du désorbeur, le solvant au moins partiellement libéré des butènes, le solvant au moins partiellement libéré des butènes étant collecté dans un collecteur de liquide du désorbeur et transporté à travers un évaporateur du désorbeur puis guidé sous le collecteur de liquide dans le fond du désorbeur, suite à quoi des butènes encore présents dans le solvant sont dégazés et le solvant étant ensuite recyclé comme flux de fond vers l'absorbeur ;
**caractérisé en ce que** la chaleur du solvant soutiré comme flux de fond du désorbeur est utilisée pour l'intégration thermique, **en ce que** la chaleur du solvant est utilisée dans à chaque fois au moins un échangeur thermique pour le préchauffage du solvant chargé transporté vers le désorbeur, pour l'évaporation dans l'évaporateur de l'absorbeur et pour l'évaporation du flux hydrocarboné liquide en C4 et **en ce que** le flux enrichi en butanes est guidé vers une oligomérisation adiabatique pour oligomériser les butènes présents dans le flux.

2. Procédé selon la revendication 1, le solvant utilisé étant de la NMP.

3. Procédé selon la revendication 1 ou 2, le solvant ou la NMP contenant de l'eau et la teneur en eau étant située entre 1 et 10% en poids, de préférence entre 4 et 9% en poids.

4. Procédé selon l'une des revendications précédentes, le flux enrichi en butènes qui est obtenu en tête du désorbeur contenant en outre de l'eau qui provient du solvant.

5. Procédé selon la revendication 4, le flux enrichi en butènes étant prélevé en tête du désorbeur et soumis à une condensation, de l'eau et un flux produit contenant des butènes se séparant par condensation et étant séparés l'un de l'autre et l'eau étant recyclée dans l'absorbeur.

6. Procédé selon la revendication 5, le flux produit contenant des butènes obtenu à partir de la condensation présentant une teneur en butènes d'au moins 70% en poids, de préférence d'au moins 75% en poids, de manière particulièrement préférée d'au moins 86% en poids par rapport à la composition totale du flux produit contenant des butènes.

7. Procédé selon l'une des revendications précédentes, la température dans le flux de fond de l'absorbeur, qui est transporté vers le désorbeur, étant 70 et 130°C, de préférence de 85 à 120°C.

8. Procédé selon l'une des revendications précédentes, la température dans le fond du désorbeur étant située entre 120 à 200°C, de préférence entre 130 à 195°C.

9. Procédé selon l'une des revendications précédentes, la pression de tête dans le désorbeur étant située entre 1 et 6 bars absolus, de préférence de 2 à 5 bars absolus.

10. Procédé selon l'une des revendications précédentes, le préchauffage du solvant chargé guidé vers le désorbeur étant effectué en deux étapes et un premier transfert de chaleur au solvant s'effectuant dans un échangeur thermique et un deuxième transfert de chaleur au solvant s'effectuant dans un évaporateur de type Kettle.

11. Procédé selon l'une des revendications précédentes, la chaleur pour l'évaporation dans l'évaporateur du désorbeur étant introduite par échange thermique dans un échangeur thermique avec un agent caloporteur approprié, en particulier de la vapeur chaude.

12. Procédé selon l'une des revendications précédentes, la vapeur chaude utilisée dans l'échangeur thermique condensant au moins partiellement et un condensat chaud étant ainsi obtenu à une pression de 10 à 20 bars absolus, de préférence de 12 à 17 bars absolus et à une température de 150 à 200°C, de préférence de 160 à 190°C, qui est alors guidé vers un récipient à condensat.

13. Procédé selon la revendication 12, une pression plus faible existant dans le récipient à condensat que dans l'échangeur thermique et une partie du condensat chaud s'évaporant ainsi de nouveau, suite à quoi toute la vapeur est obtenue sous forme de vapeur basse pression.

14. Procédé selon l'une des revendications précédentes, la vapeur chaude pour l'évaporateur du désorbeur étant mise à disposition au moyen d'un appareil à jet de vapeur dans lequel sont injectées la vapeur moyenne pression et la vapeur basse pression produite dans le récipient à condensat.
